# EUROPÄISCHE PATENTSCHRIFT

(11) **EP 2 089 074 B1**
(45) Veröffentlichungstag und Bekanntmachung des Hinweises auf die Patenterteilung: **04.07.2012**
(21) Anmeldenummer: 07819647.4
(22) Anmeldetag: 07.11.2007
(51) Int. Cl.: A61L 27/50, A61L 27/34

(54) **TEXTILE GEFÄSSPROTHESE MIT BESCHICHTUNG**
TEXTILE VASCULAR PROSTHESIS WITH A COATING
PROTHÈSE VASCULAIRE TEXTILE DOTÉE D'UN REVÊTEMENT

(30) Priorität: 13.11.2006 DE 102006053752
(43) Veröffentlichungstag der Anmeldung: 19.08.2009
(73) Patentinhaber: Aesculap AG, 78532 Tuttlingen (DE); Poly-Med, Inc., Anderson, SC 29625 (US)
(72) Erfinder: GOLDMANN, Helmut, 78532 Tuttlingen/Donau (DE); MERCKLE, Christof, 68199 Mannheim (DE); SHALABY, Shalaby W., Anderson, South Carolina 29625 (US)
(74) Vertreter: Patentanwälte Ruff, Wilhelm, Beier, Dauster & Partner
(86) Internationale Anmeldenummer: PCT/EP2007/009635
(87) Internationale Veröffentlichungsnummer: WO 2008/058660

(56) Entgegenhaltungen:
- WO-A-03/037957
- US-B1- 6 462 169
- WANG ET AL: "Evaluation of biodegradable synthetic scaffold coated on arterial prostheses implanted in rat subcutaneous tissue" BIOMATERIALS, ELSEVIER SCIENCE PUBLISHERS BV., BARKING, GB, Bd. 26, Nr. 35, 1. Dezember 2005 (2005-12-01), Seiten 7387-7401, XP005027842 ISSN: 0142-9612

## Beschreibung

Die Erfindung betrifft eine textile Gefäßprothese mit einer Beschichtung aus einem resorbierbaren Polymer in Form eines dreiarmigen Polyesters mit endständigen Hydroxylgruppen aus organischen Hydroxysäuren, die an eine zentrale trifunktionelle Hydroxyverbindung anpolymerisiert sind, wobei die drei Arme Tetrapolymere aus Lactid, ε-Caprolacton, Trimethylcarbonat und Glykolid sind.

Polymere der genannten Art und ihre Verwendung zur Beschichtung von Gefäßprothesen sind in der US 2004/0109892 A1 beschrieben. Auf den Inhalt dieser Druckschrift wird Bezug genommen. Die in dieser Druckschrift beschriebenen Polyester sind für viele Anwendungen geeignet. Bei Beschichtungspolymeren für textile Gefäßprothesen hat sich jedoch bei invivo-Versuchen gezeigt, dass unerwartete Probleme auftreten können. So ist bei einem Polymer, das eine ähnliche Zusammensetzung hat wie das in Beispiel 1 der Druckschrift beschriebene, festgestellt worden, dass sich das Beschichtungspolymer nach der Implantation einer Prothese von der Prothesenwandung ablöste und selbst nach längerer Zeit noch in benachbartem Bindegewebe auffindbar war, wo es entzündliche Reaktionen verursachte.

Aus der WO 03/037957 A1 sind absorbierbare, monozentrische, polyaxiale Copolymere mit mindestens drei Achsen bekannt. Die Achsen enthalten Lactid, ε-Caprolacton. Trimethylencarbonat und Glykolid, die an eine zentrale Hydroxyverbindung an polymerisiert sind.

Der Erfindung liegt deshalb die Aufgabe zugrunde, eine beschichtete textile Gefäßprothese zu schaffen, die ein gutes Einwachsverhalten im Körper zeigt und bei der Ablösungen und entzündliche Nebenreaktionen vermieden werden.

Diese Aufgabe wird gemäß der Erfindung bei einer Gefäßprothese der oben erwähnten Art dadurch gelöst, dass das Polymer zusammengesetzt ist aus 30 bis 45 Mol% Lactid, 20 bis 40 Mol% ε-Caprolacton, 10 bis 28 Mol% Trimethylencarbonat und 10 bis 25 Mol% Glykolid.

Intensive Versuche haben gezeigt, dass ein Beschichtungspolymer bzw. eine Imprägnierung mit der erfindungsgemäßen Zusammensetzung die an eine Beschichtung gestellten Anforderungen erfüllt. Das Polymer besitzt gute Hafteigenschaften an der textilen Prothese, so dass Ablösungen nicht zu befürchten sind. Das Beschichtungspolymer ist ausreichend flexibel, um Bewegungen der Prothese mitzumachen. Es ist trotz seiner Molekülgröße und seines sterischen Aufbaus in umweltverträglichen Beschichtungslösungsmitteln hinreichend löslich und wird in-vivo nach der Implantation ausreichend schnell abgebaut, so dass es durch das einwachsende Bindegewebe ersetzt wird.

Die trifunktionelle Hydroxyverbindung besitzt in an sich bekannter Weise vorzugsweise ein zentrales Stickstoffatom. Besonders geeignet als Trihydroxyverbindung ist Triethanolamin. Das Polymer ist bei bevorzugten Ausführungsformen ein segmentiertes Polymer aus drei mit der trifunktionellen Hydroxyverbindung verbundenen ersten Segmenten (Segment I) und drei mit den freien Enden der ersten Segmente verbundenen zweiten Segmenten (Segment II), wobei die ersten Segmente von den zweiten Segmenten verschieden sind.

Es hat sich als vorteilhaft erwiesen, wenn das erste Segment frei von Lactid ist. Vorzugsweise ist das erste Segment aus Caprolacton, Trimethylencarbonat und Glykolid gebildet.

Das zweite Segment ist vorzugsweise frei von Trimethylencarbonat. Vorteilhafterweise ist es aus Lactid, Glykolid und gegebenenfalls Caprolacton gebildet. Bei besonders bevorzugten Polymeren ist das zweite Segment frei von Caprolacton, so dass es lediglich aus Lactid und Glykolid gebildet ist.

Die Hydroxysäuren werden in an sich bekannter Weise in Form von zyklischen Monomeren eingesetzt, die unter Ringöffnung polymerisieren. Innerhalb der schon relativ engen Grenzen der Molverhältnisse der Monomere können die Eigenschaften der Copolymere, die genauer gesagt Terpolymere sind, beeinflusst werden. So enthalten bevorzugte Polymere 30 bis 40 Mol% Lactid, insbesondere 30 bis 35 Mol%. Der prozentuale Anteil an ε-Caprolacton liegt vorzugsweise bei 24 bis 40 Mol%, insbesondere 31 bis 40 Mol%. Der molare Anteil an Trimethylencarbonat liegt vorzugsweise bei 10 bis 20 Mol%, insbesondere 12 bis 16 Mol%. Der prozentuale molare Anteil an Glykolid liegt vorzugsweise bei 15 bis 20 Mol%, insbesondere 16 bis 19 Mol%. Die Polymere sind insbesondere frei von anderen Monomeren.

Auch innerhalb der Segmente sind weitere vorteilhafte Spezifizierungen möglich. So enthält das erste Segment vorzugsweise 30 bis 40, insbesondere 32 bis 35 Mol% Caprolacton, bezogen auf die Gesamtmenge der Monomere in beiden Segmenten. Der molare Anteil an Trimethylencarbonat im ersten Segment beträgt vorzugsweise 10 bis 20 Mol%, insbesondere 13 bis 17 Mol%. Der Anteil an Glykolid im ersten Segment liegt vorzugsweise bei 7 bis 12 Mol%, insbesondere 8 bis 11 Mol%, wiederum bezogen auf die Gesamtmenge der Monomeren in beiden Segmenten.

In ähnlicher Weise kann auch die molare Zusammensetzung des zweiten Segmentes spezifiziert werden. Vorzugsweise liegt der Lactidanteil im zweiten Segment bei 30 bis 45 Mol%, insbesondere 32 bis 42 Mol%. Der Lactidanteil liegt vorzugsweise in Form von L-Lactid vor. Auch DL-Lactid ist verwendbar.

In der Regel liegt das Verhältnis von Lactid zu Glykolid im zweiten Segment bei etwa 20:1 bis 4:1, wobei ein Bereich von 5:1 bis 4:1 bevorzugt ist.

Das Caprolacton liegt im zweiten Segment vorzugsweise in Mengen von 0 bis 4 Mol% vor. Der Anteil an Glykolid im zweiten Segment beträgt vorzugsweise 1 bis 10 Mol%, insbesondere 2 bis 8 Mol%.

Aufgrund des hohen Lactidanteils im zweiten Segment hat dieses einen weitgehend kristalinen Charakter und wird deshalb auch als Hartsegment bezeichnet. Demgegenüber ist das erste Segment weitgehend amorph und kann auch als Weichsegment bezeichnet werden. Variationen in den Eigenschaften der Beschichtung sind auch dadurch möglich, dass verschiedene Polymere der genannten Art in Mischung miteinander in der Beschichtung vorliegen.

Das Molekulargewicht (M_{w}) der Polymere der Beschichtung liegt in der Regel bei 100 bis 180, insbesondere bei 130 bis 150 Kilodalton. Das Zahlenmittel des Molekulargewichts (Mₙ) liegt in der Regel bei 65 bis 100, vorzugsweise bei etwa 70 bis 85 Kilodalton, insbesondere 72 bis 82 Kilodalton. Das Verhältnis M_{w}/Mₙ liegt in der Regel im Bereich von 1,0 bis 3,3, vorzugsweise im Bereich von 1,5 bis 2,1, insbesondere 1,6 bis 2,1.

Die Herstellung der Polymere für die Beschichtung kann in an sich bekannter Weise erfolgen, so wie sie in der US 2004/0109892 A1 und auch in der dort erwähnten US-Patentschrift 6,462,169 beschrieben ist.

Beschichtungslösungen aus den Polymeren können durch Auflösen der Polymere in geeigneten Lösungsmitteln hergestellt werden, wobei solche Lösungsmittel bevorzugt sind, in denen die Polymere beim Abkühlen der Lösung gelieren. Der gelierte Polymerzustand ist für die Durchführung der Beschichtung und der Erzielung von dichten Beschichtungen besonders vorteilhaft. Als Lösungsmittel sind Ketone, insbesondere 3-Pentanon, bevorzugt. Halogenierte Kohlenwasserstoffe sind nicht erforderlich, insbesondere nicht fluorierte KWS, was vorteilhaft für den Restlösungsmittelgehalt der Prothese ist.

Die Beschichtungspolymere können, falls gewünscht, biologisch aktive Wirkstoffe enthalten. Als solche kommen beispielsweise Wachstumsfaktoren und antibiotisch wirksame Substanzen, inbesondere metallisches Silber oder Silbersalze in feinster Verteilung in Frage oder antithrombogen wirkende Mittel. Derartige Zusätze sind bekannt.

Eine chemische Modifikation der Polymere selbst, insbesondere eine Vernetzung der Polymere bei der Beschichtung, ist nicht erforderlich und auch nicht gewünscht. Dadurch können die Polymere durch einfache Hydrolyse abgebaut werden.

Die textile Gefäßprothese ist vorzugsweise eine gewirkte oder gewebte Prothese, wobei die Porengröße bzw. die Durchlässigkeit der Prothesenwandung durch die Wirk- bzw. Webart und die Art der verwendeten Fäden eingestellt werden kann. Die Prothese kann in üblicher Weise eine Plissierung aufweisen. Ferner kann die Prothese mindestens eine Bifurkation haben oder mit Abzweigungen versehen sein. Bei der Gefäßprothese kann es sich auch um ein Gefäßpatch handeln, d.h. um ein flächiges Wandungsstück, das beispielsweise als Ersatz für eine defekte Gefäßwandung oder zum Verschluss einer Öffnung in einer Gefäßwandung verwendbar ist. Die Prothese kann auch mit einer externen Spirale versehen sein, die zusammen mit der Prothese beschichtet wird.

Das Material, aus dem die Prothese gefertigt ist, ist in der Regel Polyethylenterephtalat. Die verwendeten Fäden sind normalerweise multifil. Sie können als glatte und/oder als Velourfäden ausgebildet sein. Der Durchmesser der Gefäßprothesen kann zwischen 4 und 40 mm liegen. Die großen Durchmesser sind für herznahe Gefäßprothesen vorgesehen. Hier kann die Gefäßprothese auch eine dem Aortenbogen entsprechende Krümmung aufweisen.

Weitere Merkmale der Erfindung ergeben sich aus der nachfolgenden Beschreibung von bevorzugten Ausführungsformen in Verbindung mit den Unteransprüchen. Hierbei können die einzelnen Merkmale jeweils für sich alleine oder zu mehreren in Kombination bei einer Ausführungsform der Erfindung verwirklicht sein.

### Allgemeines Herstellungsbeispiel zur Synthese von kristallinen, segmentierten triaxialen Copolyestern

Zunächst erfolgt die Herstellung von amorphen, monozentrischen, triaxialen Polymerinitiatoren als Zwischenprodukte (aus trifunktioneller Hydroxylverbindung und den Monomeren von Segment I). Diese werden anschließend mit ausgewählten Mischungen der zyklischen Monomere von Segment II endgepfropft, so dass kristalline terminale Segmente II gebildet werden.

Zur Herstellung des Polymerinitiators bzw. Zwischenproduktes (Segment I) werden die Monomere Caprolacton, Trimethylencarbonat und Glykolid jeweils in Form von zyklischen Monomeren zusammen mit vorgetrocknetem Triethanolamin und Zinn(II)-2-ethylhexanoat in einen Edelstahlreaktor gegeben, der für mechanisches Rühren und Vakuum ausgerüstet ist. Der Inhalt wird eine Stunde lang bei 40°C unter Vakuum getrocknet und mit trockenem Stickstoff auf ein Druckgleichgewicht gebracht. Der Inhalt wird gerührt, um vollständiges Mischen zu gewährleisten, und die Temperatur wird auf 180 °C erhöht. Die Reaktion wird bei 180°C fortgesetzt, bis mindestens eine 50%ige, vorzugsweise eine 100%ige Monomerumsetzung erreicht ist (durch High Pressure Liquid Chromatography - HPLC, oder durch Gaschromatographie - GC bestimmt). Der Polymerinitiator wird dann entweder abgekühlt und etwa 15 Stunden lang auf Raumtemperatur gehalten oder auf der Temperatur von 180 °C bis zum nächsten Schritt gehalten.

Der Polymerinitiator wird dann auf 110 °C erwärmt bzw. abgekühlt und die monomeren Komponenten von Segment II (Lactid, gegebenenfalls Caprolacton, und Glykolid) werden in den jeweiligen Mengen hinzugefügt. Die Mischung wird gerührt, bis der Polymerinitiator gelöst ist. Dann wird die Temperatur auf 160 °C erhöht. Bei Polymeren mit einem Molverhältnis von Gesamtmonomer/Katalysator von 28.000 wird eine weitere gleiche Menge an Zinn(II)-2-ethylhexanoat hinzugegeben, bevor die Temperatur auf 160 °C erhöht wird. Der Inhalt wird noch ca. 30 Minuten lang gerührt, nachdem die Temperatur 160 °C erreicht hat. Das Rühren wird dann gestoppt und die Reaktion zur Bildung von Segment II durch Erwärmen auf 130 bis 150 °C fortgesetzt bis praktisch vollständige Umsetzung erreicht ist (durch HPLC oder GC bestimmt).

Nach Beendigung der Polymerisation wird das Polymer abgelassen und in etwa 2,5 cm große Stücke zerkleinert. Restliche Monomere werden durch Destillation unter reduziertem Druck bei Temperaturen bis 80 °C abgezogen. Das Polymer wird dann weiter gereinigt durch Ausfällung aus einer Lösung von 20% (Gew./Vol.) in Dichlormethan (DCM) unter Verwendung von 2 Volumenteilen Isopropylalkohol (IPA) bei -60 °C in einem handelsüblichen Mischer. Das gereinigte Polymer wird dann bei Temperaturen bis 80 °C unter reduziertem Druck auf ein konstantes Gewicht getrocknet. Eine weitere Reinigung wird durch Ausfällen des gereinigten Polymers aus einer konzentrierten Acetonlösung durch ein Gießen in 2-Propanol vorgenommen. Daraus wird ein trockenes Produkt isoliert.

### Beschichtung einer Gefäßprothese

Eine Gefäßprothese, die mit einer Plissierung versehen ist, wird auf einem Stab ausgezogen und längsfixiert, so dass die Plissierung im wesentlichen aufgehoben ist.

Eine geeignete Menge des Beschichtungspolymers wird in 3-Pentanon bei ca. 80 °C gelöst, wobei eine etwa 10%ige (Gew./Vol.) Lösung hergestellt wird. Man lässt die Lösung abkühlen, wobei das Polymer einen Gelzustand annimmt. In dieses Gel wird der Stab mit der aufgespannten Gefäßprothese mehrfach eingetaucht. Zwischen den Tauchvorgängen lässt man überschüssiges Gel von der Prothese abtropfen, wobei die Prothese gleichzeitig bewegt wird, um eine gleichmäßige Beschichtung zu erhalten. Zwischen den jeweiligen Tauchvorgängen wird die Prothese jeweils 2 Stunden bei Raumtemperatur getrocknet. Danach wird die Beschichtung auf der Gefäßprothese getrocknet und bei 60 °C fixiert.

Bei der Beschichtung gibt es zahlreiche Variationsmöglichkeiten. So kann die Beschichtungslösung auch aufgestrichen oder aufgesprüht werden. Die Beschichtungslösung kann auch eingewalkt werden. Ferner kann die Prothese vor der Beschichtung oder zwischen einzelnen Beschichtungsvorgängen gewendet werden, so dass die Protheseninnenwandung nach außen zu liegen kommt.

Auf diese Weise kann die Prothese an ihrer Außenwandung, an ihrer Innenwandung, innen und außen sowie auch im Inneren der Wandung beschichtet bzw. imprägniert werden, je nachdem welche Beschichtungsart bevorzugt ist.

Im trockenen Zustand ist die Prothesenwandung durch die filmbildende Beschichtung gut abgedichtet. Die Beschichtung haftet gut an der Prothese und kann Bewegungen der Prothese ohne Bruch mitmachen. Irgendwelche Weichmacher sind nicht erforderlich.

Die Erfindung wird durch die nachfolgenden Beispiele und die zugehörige Beschreibung noch weiter erläutert.

### Beispiele 1 bis 8

### Polymerzusammensetzung

In den Tabellen 1-3 sind die Polymerzusammensetzungen sowie die physikalischen Daten der Polymere nach den Beispielen 1 bis 8, wie ein Polymer ähnlich dem Beispiel 1 der US 2004/0109892 A1 (Vergleichsbeispiel) gegenübergestellt:

**Tabelle 1: Polymerzusammensetzung**

| **Polymercharge** | **Zusammensetzung [Mol-%]** | | | |
|---|---|---|---|---|
| | **L-Lactid** | **ε-CL** | **TMC** | **Glykolid** |
| **Vergleichsbeispiel** | | | | |
| **ähnlich Stand der Technik** | 40 | 30 | 26 | 4 |
| **Beispiel 1** | 36 | 24 | 26 | 14 |
| **Beispiel 2** | 36 (D,L-) | 24 | 26 | 14 |
| **Beispiel 3** | 36 | 34 | 16 | 14 |
| **Beispiel 4** | 42 | 32 | 16 | 10 |
| **Beispiel 5:** | 39 | 28 | 21 | 12 |
| **Mischung von 1 und 4 (50:50)** | | | | |
| **Beispiel 6** | 34 | 35 | 14 | 17 |
| **Beispiel 7** | 32 | 35 | 14 | 19 |
| **Beispiel 8** | 33 | 35 | 14 | 18 |

Die Polymere unterscheiden sich in ihrer Gesamtzusammensetzung, vor allem hinsichtlich der Anteile an Glykolid und Lactid.

In der zweiten Polymerserie wurde der Glykolid-Anteil ausgehend vom 4 % auf das Maximum von 19 % gesteigert und der L-Lactid von 40 auf 32 % gesenkt.

In Tabelle 2 sind die Komponenten der zwei Segmente aus dem das RandomPolymer besteht zusammengestellt:

**Tabelle 2: Polymerzusammensetzung in den Segmenten 1 und 2**

| **Polymercharge** | **Zusammensetzung (Mol-%)** | |
|---|---|---|
| | **Segment 1 (weich)** | **Segment 2 (hart)** |
| | CL/TMC/G | LL/CL/G |
| **Vergleichsbeispiel** | 26/26/4 | 40/4/0 |
| **Beispiel 1** | 22/26/8 | 36/2/6 |
| **Beispiel 2** | 22/26/8 | 36 (D,L-)/2/6 |
| **Beispiel 3** | 32/16/8 | 36/2/6 |
| **Beispiel 4** | 32/16/8 | 42/0/2 |
| **Beispiel 5 (50:50)** | 27/21/8 | 39/1/4 |
| **Beispiel 6** | 35/14/9 | 34/0/8 |
| **Beispiel 7** | 35/14/11 | 32/0/8 |
| **Beispiel 8** | 35/14/10 | 33/0/8 |

In Segment 2 wurde der Glykolid-Anteil von 0 auf 8 % angehoben und im Segment 1 von 4 auf maximal 11 %. Die Beispiele 6 bis 8 betreffen besonders bevorzugte Polymerzusammensetzungen.

In Tabelle 3 sind die physikalischen Daten der Beschichtungspolymere aufgeführt.

**Tabelle 3: Physikalische Daten**

| **Polymercharge** | **Schmelz-temp.** | **T_{G}** | **Schm elzwä rme** | **inh. Visko sit.** | **Molekulargewicht** | | |
|---|---|---|---|---|---|---|---|
| | **[°C]** | **[°C ]** | **[J/g]** | **[dl/g]** | **M_{w} [kDa]** | **Mₙ [kDa]** | **M_{w}/mₙ** |
| **Vergleichsbeispiel** | | | | | | | |
| | 148 | -37 | 13.1 | 1.32 | 137 | 82 ±9 | 1.66 |
| **Beispiel 1** | - | -3 | - | 1.16 | 132 | 72 | 1.83 |
| **Beispiel 2** | - | -4 | - | 1.23 | 137 | 73 | 1.88 |
| **Beispiel 3** | 121 | -28 | 7.9 | 1.45 | 144 | 82 | 1.76 |
| **Beispiel 4** | 149 | -37 | 15.8 | 1.49 | 142 | 81 | 1.75 |
| **Beispiel 5 (50:50)** | 147 | -12 | 7.2 | 1.43 | 137 | 76 | 1.80 |
| **Beispiel 6** | 109 | -29 | 7.4 | 1.45 | 146 | 82 | 1.78 |
| **Beispiel 7** | 101 | -26 | 5.1 | 1.23 | 135 | 74 | 1.82 |
| **Beispiel 8** | 106 | -22 | 4.1 | 1.15 | 134 | 73 | 1.84 |

Die inhärente Viskosität wurde bei 25 °C in Chloroform mit 200 mg Probe/100 ml via Kapillare OC gemessen.

Das Polymer wird bei 80 °C in 3-Pentanon zu einer 10 %igen Lösung gelöst und bei 5 °C über 24 Stunden ausgelieren gelassen.

Die Beschichtung erfolgt mit plissierten 8 mm Gefäßprothesen und einer Auslängung von 22 cm beim Beschichten mit auf PE-Stab aufgezogenen Prothesen.

Beschichtungen mit frei hängenden Prothesen werden mit plissierten Prothesen durchgeführt, die mit einem 62 g Gewicht beschwert auf eine Länge von 25 cm zurechtgeschnitten wurden.

Die Gestelle werden in beiden Fällen entsprechend der Angaben in der Tabelle mehrmalig in die Beschichtungslösung getaucht.

Die Beschichtungsparameter ergeben sich aus Tabelle 4.

**Tabelle 4: Beschichtungsparameter**

| **Polymer** | **Temperatur** | **Halterung** | **Tauchgänge** | **Verweilzeit in Beschichtungslösung** | **Beschichtungsgehalt** |
|---|---|---|---|---|---|
| | **[°C]** | | **[Anzahl]** | **[s]** | **[%]** |
| **Vergleichsbeispiel** | 50 | PE-Stab | 3 | 3 | 45 |
| | 67 | freihängend | 3 | 30 | 56 |
| **Beispiel 3** | 35 | PE-Stab | 3 | 3 | 47 |
| | 50 | freihängend | 3 | 3 | 57 |
| **Beispiel 4** | 75 | PE-Stab | 4 | 3 | 48 |
| **Beispiel 6** | 40 | PE-Stab | 3 | 3 | 49 |
| | 30 | PE-Stab | 3 | 3 | 50 |
| | 30 | PE-Stab | 4 | 3 | 57 |
| | 30 | freihängend | 3 | 3 | 55 |
| **Beispiel 7** | 30 | PE-Stab | 3 | 3 | 47 |
| | 30 | PE-Stab | 3 | 3 | 47 |
| | 30 | PE-Stab | 4 | 3 | 55 |
| | Gelier.: 24 h bei 5 °C | freihängend | 4 | 3 | 61 |
| | Besch.: 30 °C | | | | |
| **Beispiel 8** | 1x 50 °C; 5 °C | PE-Stab | 4 | 1 x 30 , 3x3 | 56 |
| | Gelier.: 0 °C Besch.: 25 °C | PE-Stab | 4 | 3 | 52 |
| | Gelier.: 24 h bei 5 °C Besch.: 25 °C | PE-Stab | 3 | 3 | 51 |
| | Gelier.: 24 h bei 5 °C Besch.: 25 °C | PE-Stab | 4 | 3 | 58 |
| | Gelier.: 24 h bei 5 °C Besch.: 25 °C | PE-Stab | 3 | 3 | 53 |
| | Gelier.: 24 h bei 5 °C Besch.: 25 °C | PE-Stab | 4 | 3 | 59 |

Ein Beschichtungsgradient auf der Prothese wird durch das stete Wenden der Prothesen beim Trocknen verhindert. Darüber hinaus werden die Tauchzyklen mit jeweils gewendeten Prothesen und/oder bezogen auf die Eintauchrichtung gedrehten durchgeführt.

Im Anschluss an die Trocknung werden die Prothesen auf 12 cm aufgeschoben und 30 min. bei 60 °C fixiert und getrocknet. Nach dem Verpacken und einer Ethylenoxid (EO)-Sterilisation der Prothesen durch die gasdurchlässige und bakteriendichte Verpackung hindurch (Tyvek^{®}-Deckfolien) werden die verpackten Prothesen bei 50 °C über 24 Stunden mit Vakuumzyklen und trockener Luft (H₂O-Gehalt: <1 ppm) abschließend getrocknet und mit einer Dampfbarriere durch eine Alu/Papier/PE-Deckfolie final versiegelt.

**Tabelle 5: Daten der beschleunigten Polymerabbaustudie (in vitro)**

| | **Verbleibende Beschichtung nach [%]** | | | |
|---|---|---|---|---|
| **Polymer** | **22 Tage** | **33 Tage** | **42 Tage** | **54 Tage** |
| **Vergleichsbeispiel** | 97 | 95 | 94 | 92 |
| **Beispiel 3** | 94 | 91 | 85 | 75 |
| **Beispiel 4** | 95 | 91 | 88 | 83 |
| **Beispiel 6** | 94 | 87 | 81 | 59 |
| **Beispiel 7** | 92 | 85 | 78 | 55 |
| **Beispiel 8** | 93 | 84 | 67 | 29 |

### Degradationsstudien (in vitro)

Diese Studie wurde mit Prüfkörpern (∅: 0.5 in., Dicke: 0.07 in.) in Phosphatpuffer (pH = 12) bei 37 °C durchgeführt.

Der Polymerabbau des Polymer nach Vergleichsbeispiel war mit einem Abbau über 56 Tage bis auf 92% der ursprünglichen Polymermasse deutlich langsamer als der der anderen Polymere.

Die Polymere der Beispiele 1 bis 8 waren innerhalb der ersten 54 Tage maximal bis auf 29 % abgebaut.

### In-vivo-Degradationsstudien

Die Resorptionszeiten von mit dem Vergleichspolymer beschichteten Prothesen aus der funktionellen Langzeittierstudie im Schaf von einem Jahr, zeigte bei explantierten Prothesen noch nach 12 Monaten Polymerreste, die entzündliche Gewebereaktionen hervorriefen.

Die mit den beschichteten Prothesen durchgeführten Degradationsstudien in einer Kleintierstudie "Ratte" bestätigen die Degradationsergebnisse der Polymere aus dem beschleunigten In-vitro-Versuch. So sind nach 4 Wochen vom Polymer nach Beispiel 8 keine Beschichtungs-Residuen mehr nachweisbar. Bei Beispiel 6 finden sich wie bei Beispiel 7 nur noch minimale Reste der Beschichtung. Ablösungen der Beschichtung wurden bei den Polymeren nach Beispiel 1 bis 8 nicht beobachtet.

**Tabelle 6: In-vivo Polymerabbau nach 4 Wochen subkutan in der Ratte.**

| **Polymer** | **Residuen semiqantitativ** | **Standardabweichung** |
|---|---|---|
| Beispiel 6 | 0,17 | 0,4 |
| Beispiel 7 | 1 | 0 |
| Beispiel 8 | 0 | 0 |
| Vergleichsbeispiel | 2,2 | 0,4 |

## Patentansprüche

1. Textile Gefäßprothese mit einer Beschichtung aus einem resorbierbaren Polymer in Form eines dreiarmigen Polyesters mit endständigen Hydroxylgruppen aus Hydroxysäuren, die an eine zentrale trifunktionelle Hydroxyverbindung anpolymerisiert sind, wobei die drei Arme Tetrapolymere aus Lactid, ε-Caprolacton, Trimethylencarbonat und Glykolid sind, **dadurch gekennzeichnet, dass** das Polymer zusammengesetzt ist aus 30 bis 45 Mol% Lactid, 20 bis 40 Mol% ε-Caprolacton, 10 bis 28 Mol% Trimethylencarbonat und 10 bis 25 Mol% Glykolid.

2. Gefäßprothese nach Anspruch 1, **dadurch gekennzeichnet, dass** das Polymer ein segmentiertes Polymer aus drei mit der trifunktionellen Hydroxyverbindung verbundenen ersten Segmenten und drei mit den freien Enden der ersten Segmente verbundenen zweiten Segmenten ist, wobei die ersten Segmente von den zweiten Segmenten verschieden sind.

3. Gefäßprothese nach Anspruch 1 oder 2, **dadurch gekennzeichnet, dass** das erste Segment frei von Lactid ist.

4. Gefäßprothese nach einem der vorhergehenden Ansprüche, **dadurch gekennzeichnet, dass** das zweite Segment frei von Trimethylencarbonat ist.

5. Gefäßprothese nach einem der vorhergehenden Ansprüche, **dadurch gekennzeichnet, dass** das erste Segment aus Caprolacton, Trimethylencarbonat und Glykolid besteht.

6. Gefäßprothese nach einem der vorhergehenden Ansprüche, **dadurch gekennzeichnet, dass** das zweite Segment aus Lactid, Glykolid und gegebenenfalls Caprolacton besteht.

7. Gefäßprothese nach einem der vorhergehenden Ansprüche, **dadurch gekennzeichnet, dass** das zweite Segment frei von Caprolacton ist.

8. Gefäßprothese nach einem der vorhergehenden Ansprüche, **dadurch gekennzeichnet, dass** das erste Segment 30 bis 40, insbesondere 32 bis 35 Mol% Caprolacton, bezogen auf die Gesamtmenge der Monomere in beiden Segmenten enthält.

9. Gefäßprothese nach einem der vorhergehenden Ansprüche, **dadurch gekennzeichnet, dass** das erste Segment 10 bis 20 Mol%, insbesondere 13 bis 17 Mol% Trimethylencarbonat, bezogen auf die Gesamtmenge der Monomeren in den beiden Segmenten enthält.

10. Gefäßprothese nach einem der vorhergehenden Ansprüche, **dadurch gekennzeichnet, dass** das erste Segment 7 bis 12 Mol%, insbesondere 8 bis 11 Mol% Glykolid, bezogen auf die Gesamtmenge der Monomeren in beiden Segmenten enthält.

11. Gefäßprothese nach einem der vorhergehenden Ansprüche, **dadurch gekennzeichnet, dass** das zweite Segment 30 bis 45 Mol%, insbesondere 32 bis 42 Mol%, Lactid, bezogen auf die Gesamtmenge der Monomeren in den beiden Segmenten enthält.

12. Gefäßprothese nach einem der vorhergehenden Ansprüche, **dadurch gekennzeichnet, dass** das zweite Segment 0 bis 4 Mol% Caprolacton, bezogen auf die Gesamtmenge der Monomere in beiden Segmenten enthält.

13. Gefäßprothese nach einem der vorhergehenden Ansprüche, **dadurch gekennzeichnet, dass** das zweite Segment 1 bis 10 Mol%, insbesondere 2 bis 8 Mol% Glykolid, bezogen auf die Gesamtmenge der Monomeren in den beiden Segmenten enthält.

14. Gefäßprothese, **dadurch gekennzeichnet, dass** die Beschichtung in Form einer Mischung aus mindestens zwei verschiedenen Polymeren nach einem der vorhergehenden Ansprüche zusammengesetzt ist.

## Claims

1. Textile vascular prosthesis having a coating composed of an absorbable polymer in the form of a three-armed polyester having terminal hydroxyl groups from hydroxy acids polymerized onto a central trifunctional hydroxy compound, the three arms being tetrapolymers of lactide, ε-caprolactone, trimethylene carbonate and glycolide, **characterized in that** the polymer is composed of 30 to 45 mol% of lactide, 20 to 40 mol% of ε-caprolactone, 10 to 28 mol% of trimethylene carbonate and 10 to 25 mol% of glycolide.

2. Vascular prosthesis according to Claim 1, **characterized in that** the polymer is a segmented polymer comprising three first segments connected by the trifunctional hydroxy compound and three second segments connected to the free ends of the first segments, the first segments differing from the second segments.

3. Vascular prosthesis according to Claim 1 or 2, **characterized in that** the first segment is free of lactide.

4. Vascular prosthesis according to any one of the preceding claims, **characterized in that** the second segment is free of trimethylene carbonate.

5. Vascular prosthesis according to any one of the preceding claims, **characterized in that** the first segment consists of caprolactone, trimethylene carbonate and glycolide.

6. Vascular prosthesis according to any one of the preceding claims, **characterized in that** the second segment consists of lactide, glycolide and optionally caprolactone.

7. Vascular prosthesis according to any one of the preceding claims, **characterized in that** the second segment is free of caprolactone.

8. Vascular prosthesis according to any one of the preceding claims, **characterized in that** the first segment contains 30 to 40, particularly 32 to 35 mol% of caprolactone, based on the total amount of the monomers in the two segments.

9. Vascular prosthesis according to any one of the preceding claims, **characterized in that** the first segment contains 10 to 20 mol%, particularly 13 to 17 mol% of trimethylene carbonate, based on the total amount of the monomers in the two segments.

10. Vascular prosthesis according to any one of the preceding claims, **characterized in that** the first segment contains 7 to 12 mol%, particularly 8 to 11 mol% of glycolide, based on the total amount of the monomers in the two segments.

11. Vascular prosthesis according to any one of the preceding claims, **characterized in that** the second segment contains 30 to 45 mol%, particularly 32 to 42 mol% of lactide, based on the total amount of the monomers in the two segments.

12. Vascular prosthesis according to any one of the preceding claims, **characterized in that** the second segment contains 0 to 4 mol% of caprolactone, based on the total amount of the monomers in the two segments.

13. Vascular prosthesis according to any one of the preceding claims, **characterized in that** the second segment contains 1 to 10 mol%, particularly 2 to 8 mol% of glycolide, based on the total amount of the monomers in the two segments.

14. Vascular prosthesis, **characterized in that** the coating is composed in the form of a mixture of at least two different polymers according to any one of the preceding claims.

## Revendications

1. Prothèse vasculaire textile comportant un revêtement à base d'un polymère résorbable sous forme d'un polyester à trois bras, comportant des groupes hydroxy terminaux d'acides hydroxylés qui sont attachés par polymérisation à un composé hydroxylé trifonctionnel central, les trois bras étant des tétrapolymères de lactide, ε-caprolactone, carbonate de triméthylène et glycolide, **caractérisée en ce que** le polymère est composé de 30 à 45 % en moles de lactide, 20 à 40 % en moles d'ε-caprolactone, 10 à 28 % en moles de carbonate de triméthylène et 10 à 25 % en moles de glycolide.

2. Prothèse vasculaire selon la revendication 1, **caractérisée en ce que** le polymère est un polymère segmenté constitué de trois premiers segments liés au composé hydroxylé trifonctionnel et trois seconds segments liés aux extrémités libres des premiers segments, les premiers segments étant différents des seconds segments.

3. Prothèse vasculaire selon la revendication 1 ou 2, **caractérisée en ce que** le premier segment est exempt de lactide.

4. Prothèse vasculaire selon l'une quelconque des revendications précédentes, **caractérisée en ce que** le second segment est exempt de carbonate de triméthylène.

5. Prothèse vasculaire selon l'une quelconque des revendications précédentes, **caractérisée en ce que** le premier segment est constitué de caprolactone, carbonate de triméthylène et glycolide.

6. Prothèse vasculaire selon l'une quelconque des revendications précédentes, **caractérisée en ce que** le second segment est constitué de lactide, glycolide et éventuellement caprolactone.

7. Prothèse vasculaire selon l'une quelconque des revendications précédentes, **caractérisée en ce que** le second segment est exempt de caprolactone.

8. Prothèse vasculaire selon l'une quelconque des revendications précédentes, **caractérisée en ce que** le premier segment contient 30 à 40, en particulier 32 à 35 % en moles de caprolactone, par rapport à la quantité totale des monomères dans les deux segments.

9. Prothèse vasculaire selon l'une quelconque des revendications précédentes, **caractérisée en ce que** le premier segment contient 10 à 20 % en moles, en particulier 13 à 17 % en moles de carbonate de triméthylène, par rapport à la quantité totale des monomères dans les deux segments.

10. Prothèse vasculaire selon l'une quelconque des revendications précédentes, **caractérisée en ce que** le premier segment contient 7 à 12 % en moles, en particulier 8 à 11 % en moles de glycolide, par rapport à la quantité totale des monomères dans les deux segments.

11. Prothèse vasculaire selon l'une quelconque des revendications précédentes, **caractérisée en ce que** le second segment contient 30 à 45 % en moles, en particulier 32 à 42 % en moles, de lactide, par rapport à la quantité totale des monomères dans les deux segments.

12. Prothèse vasculaire selon l'une quelconque des revendications précédentes, **caractérisée en ce que** le second segment contient 0 à 4 % en moles de caprolactone, par rapport à la quantité totale des monomères dans les deux segments.

13. Prothèse vasculaire selon l'une quelconque des revendications précédentes, **caractérisée en ce que** le second segment contient 1 à 10 % en moles, en particulier 2 à 8 % en moles de glycolide, par rapport à la quantité totale des monomères dans les deux segments.

14. Prothèse vasculaire, **caractérisée en ce que** le revêtement est composé sous forme d'un mélange d'au moins deux différents polymères selon l'une quelconque des revendications précédentes.
